# EUROPEAN PATENT SPECIFICATION

(11) **EP 0 707 611 B1**
(45) Date of publication and mention of the grant of the patent: **10.09.1997**
(21) Application number: 94923833.1
(22) Date of filing: 04.07.1994
(51) Int. Cl.: C08G 73/02

(54) **PROCESS FOR THE PREPARATION OF A DENDRITIC MACROMOLECULE**
VERFAHREN ZUR HERSTELLUNG EINES DENDRITISCHEN MAKROMOLEKÜLS
PROCEDE DE PREPARATION D'UNE MACROMOLECULE DENDRITIQUE

(30) Priority: 08.07.1993 BE 9300702
(43) Date of publication of application: 24.04.1996
(73) Proprietor: DSM N.V., 6411 TE Heerlen (NL)
(72) Inventor: DE BRABANDER-VAN DEN BERG, Ellen, Marleen, Monique, NL-6166 HX Geleen (NL); CASTELIJNS, Anna, Maria, Cornelia, Francisca, NL-6176 JB Beek (NL); DE MAN, Hendrikus, Cornelis, Johannes, NL-6165 CP Geleen (NL); REINTJENS, Rafael, Wilhelmus, Elisabeth, Ghislain, NL-6333 BP Nuth (NL)
(86) International application number: NL9400152
(87) International publication number: WO9502008

(56) References cited:
- WO-A-93/14147
- DE-A- 2 739 917
- DE-A- 3 248 326

## Description

The invention relates to a process for the preparation of a dendritic macromolecule, wherein an amount of a core molecule comprising at least one functional group is dissolved in a solvent, after which alternatingly an addition reaction and a hydrogenation reaction are carried out, during the addition reaction vinyl cyanide units being added to the solution which react with the functional groups in such a manner that a dendritic macromolecule with terminal cyanide groups is formed, and during the hydrogenation reaction the cyanide groups being reduced in solution, by means of hydrogen and a suitable catalyst in such a manner that functional amine groups are formed.

WO-A-9314147 describes a process wherein a diamine, for instance 1,4-diaminobutane, is dissolved in methanol, after which a vinyl cyanide, for instance acrylonitrile, is added. When the Michael addition reaction has taken place the excess of vinyl cyanide is distilled off, after which a cyanide-terminated reaction product is obtained. Next, this cyanide-terminated reaction product is reduced in water by means of hydrogen. The amine-terminated reaction product thus obtained is then isolated by evaporation of the water. The two reactions are carried out alternatingly, so that a dendritic macromolecule of the desired generation is obtained.

The process described in WO-A-9314147 has the disadvantage that the concentration of cyanide-terminated product during the hydrogenation reaction is relatively low. On the one hand this means that much solvent has to be removed after the hydrogenation reaction, while on the other hand the capacity of the reactors is not utilized effectively. Although the process can be readily scaled up and for that reason among others is eminently suitable for the preparation of dendritic macromolecules on a commercial scale, it is disadvantageous from the above-mentioned points of view. An additional disadvantage is that relatively much catalyst has to be added in the hydrogenation reaction. Another additional disadvantage is the foaming that occurs when the solvent is distilled off upon completion of the hydrogenation reaction and when the reactor is opened after completion of the hydrogenation reaction.

It is the aim of the present invention to provide a process in which the above-mentioned disadvantages do not occur or occur to a significantly lesser extent.

The invention is characterized in that the solvent in which the hydrogenation reaction takes place is an alcohol which contains an amount of ammonia, the molar ratio between the amount of ammonia and the number of cyanide groups being higher than 0.8.

Mostly the molar ratio between the amount of ammonia and the number of cyanide groups is lower than 50. Mostly a large portion of the ammonia will be present in the gas phase; only a fraction dissolves in the alcohol. The molar ratio between the amount of ammonia and the number of cyanide groups preferably is higher than 1 and lower than 20.

Surprisingly, it has been found that with the process according to the invention the capacity of the reactors for the hydrogenation reactions can be utilized considerably more effectively. It has also been found that the required amount of catalyst in the process according to the invention is considerably smaller and that the selectivity of the reactions involved is further enhanced with the process according to the invention. In addition it has appeared that less foaming or no foaming at all occurs during the hydrogenation reaction and when the reactor is opened after completion of the hydrogenation reaction.

In Angew. Chem. Int. Ed. Engl. 29 (1990), pp. 138-175, several possible applications of dendritic macromolecules are mentioned, such as the use of dendritic macromolecules for the calibration of sieves, as catalyst (carriers), as selective membranes, the use of dendritic macromolecules for electronic purposes and in coatings, but the use of dendritic macromolecules as impact modifiers or as cross-linking agents in various plastics is also conceivable.

In the process according to the invention an amount of the core molecule is dissolved in a solvent.

The molecules that can be used as core molecules according to the present invention are molecules which contain at least one functional group. In the framework of the present invention a functional group is a group which can react - optionally in the presence of a suitable catalyst - with a vinyl cyanide unit.

Groups that can react with a vinyl cyanide unit under favourable reaction conditions are for instance hydroxyl groups, primary and secondary amine groups, thiol groups, carbon compounds with electronegative substituents, such as an ester group, an amide group, a ketone group, an aldehyde group, a carboxylic acid group and salts thereof. Preferably, the core molecule contains a hydroxyl group, a primary amine group and/or a secondary amine group as functional group.

Depending on the nature of the functional group, it can react with one or more vinyl cyanide units. If a functional group can react with F vinyl cyanide units, its functionality is F. A hydroxyl group can react with one vinyl cyanide unit and thus its functionality F is 1. A primary amine group can react with two vinyl cyanide units and thus its functionality F is 2. Mostly the functionality F is 1, 2 or 3.

Mostly, each functional group of the core molecule having a functionality F is reacted with F vinyl cyanide units.

The vinyl cyanide units contain a double bond as well as an electron-withdrawing group which is conjugated with the double bond. The vinyl cyanide units are chosen from the group of compounds according to formula 1: 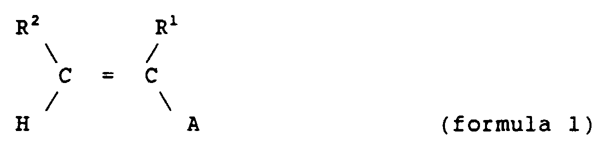 where
- R¹ =: -H or -CH₃;
- R² =: -H, -CH₃, or a hydrocarbon compound having 2-18 carbon atoms and containing at least one double bond conjugated with the double bond of formula 1 such as for example -CH=CH₂, -CH=C-CH=CH₂;
- A =: -C≡N, 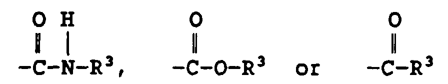
- R³ =: a hydrocarbon compound having 1-18 carbon atoms and containing 1-5 cyanide groups;

Particularly suitable vinyl cyanide units that can be used are acrylonitrile and methacrylonitrile (MACN).

In a first preferred embodiment of the invention the core molecule preferably contains 1-10 functional groups. Suitable core molecules can for instance be chosen from the group of ammonia, water, methanol, polymethylene diamines, such as hexamethylene diamine, ethylene diamine and 1,4-diaminobutane (DAB), diethylene triamine, triethylene tetramine, tetraethylene pentamine, linear and branched polyethyleneimine, methylamine, hydroxyethylamine, octadecylamine, polyaminoalkylarenes, such as 1,3,5-tris(aminoethyl)benzene, tris(aminoalkyl)amines, such as tris(aminoethyl)amine, heterocyclic amines, such as imidazolines and piperidines, hydroxyethylaminoethyl amine, mercaptoethyl amine, morpholine, piperazine, pentaerythritol, polyalkylene polyols, such as polyethylene glycol and polypropylene glycol, glycols, such as ethylene glycol, polyalkylene polymercaptans, 1,2-dimercaptoethane, phosphine, ε-aminocapronic acid, glycine, thiophenols, phenols, melamine and derivatives thereof, such as melamine tris(hexamethylenediamine). Preferably, a core molecule chosen from the group of methylene diamines, glycols and tris(1,3,5-aminomethyl)benzene is used in the process according to the invention. More preferably, 1,4-diaminobutane is used as core molecule.

According to a second preferred embodiment of the invention a (co)polymer containing one or more of the above-mentioned functional groups is used as core of the dendritic macromolecule. Examples of such (co)polymers are styrene-maleimide copolymers, styrene-acrylonitrile copolymers, polyethyleneimine and polymers such as for instance polypropylene oxide, polystyrene and ethylene-propylene-diene copolymers, which are functionalized with one or more of the above-mentioned functional groups, for instance NH₂ groups.

According to a third preferred embodiment of the invention, dendrimers of a low generation, for instance 1st, 2nd or 3rd generation, described in US-A-4507466 and by F. Vögtle et al., Synthesis, February 1978, pp. 155-158, are used as core of the dendritic macromolecule. Particularly in this case the functionality of the core molecule can be very high; for instance 10-40 amine groups can be present. The molecular weight of such core molecules is mostly higher than 200 and mostly lower than 5000.

The shape of the dendritic macromolecule is to a large extent determined by the shape of the core molecule chosen. If a small molecule or a spherical dendrimer is used as core molecule, a spherical dendritic macromolecule can be obtained. If a polymer that only has terminal functional groups is used as core molecule, the dendritic macromolecule obtained has a more elongated shape.

A process for the preparation of the dendritic macromolecule via the Michael addition comprises the following steps:
a) virtually all functional groups of the core molecule are reacted with one or more vinyl cyanide units;
b) virtually all incorporated vinyl cyanide units are hydrogenated to amine groups;
c) virtually all amine groups thus formed are reacted with vinyl cyanide units;
steps b) and c) being carried out alternatingly (N-1) times in order to obtain a macromolecule of the desired generation N. The value of N mostly varies from 1 to 10; N preferably has a value of 2 or higher, in particular 3 or higher.

In order to obtain a dendritic macromolecule of a specific generation the above addition and hydrogenation reactions are repeated alternatingly a number of times. After one addition reaction a molecule of the first generation is obtained. When three addition reactions and two hydrogenation reactions have been carried out alternatingly a dendritic macromolecule of the third generation is obtained.

The process of preparation can be stopped after a reaction step b), in which case a dendritic macromolecule of generation 1.5, 2.5 or higher is obtained.

In the process according to the invention it is not necessary each time to purify the product obtained in the separate intermediate steps. But mostly the product obtained in the separate intermediate steps is isolated, in the sense that the excess of reagents, the catalyst and the solvent are removed.

A number of branches, made from vinyl cyanide units, emanate from the dendritic macromolecule thus obtained. If the reactions involved run to completion, the total number of branches of the desired generation N can be calculated as follows. If G is the number of functional groups of the core molecule and F the functionality of each separate functional group, the number of reactive sites R of the core molecule is equal to the sum of the functionalities F of all functional groups G. The maximum number of branches of the N^{th} generation can be described as the number of reactive sites R multiplied by F^{N-1}. If the reactions involved do not run to completion, the number of branches is smaller than R multiplied by F^{N-1}.

The Michael addition is mostly carried out in solution. To that end an amount of the core molecule or an amount of a dendritic macromolecule of generation N + 0.5 is dissolved in a solvent. Preferably such a solvent is chosen as to produce a maximum favourable effect on both the course of the reactions involved and the prevention of side reactions. This implies that it is important to choose a solvent which under the reaction conditions applied does not react with the functional groups of the core molecule or the dendritic macromolecule of generation N + 0.5. The ultimate choice of the solvent depends to a large extent on the nature of the functional groups of the core molecule or the dendritic macromolecule of generation N + 0.5.

Suitable solvents can be selected for instance from the group consisting of water, terahydrofuran, various alcohols, such as for instance methanol, ethanol, isopropanol and mixtures of these solvents. Preferably, water, methanol or a mixture of these is used. More preferably, water is used as solvent.

Next, vinyl cyanide units are added to the solution thus obtained of the core molecule or dendritic macromolecule of generation N + 0.5 in the solvent. It is therefore of importance as well that the solvent does not react with the vinyl cyanide units.

If it is desired in this reaction step to have virtually each reactive site of the core react with a vinyl cyanide unit, then the ratio between the reactants, which can be described as the ratio between the number of vinyl cyanide units and the number of reactive sites, should be at least 1. If this ratio is lower than 1, not each reactive site will react with a vinyl cyanide unit. The ratio between the number of vinyl cyanide units and the number of reactive sites is mostly between 1 and 5, preferably between 1 and 2.5.

In the process described in WO-A-9314147 the vinyl cyanide is added to a solution of the substrate in the solvent slowly. The reaction mixture is cooled continuously to avoid undesired side reactions. According to WO-A-9314147 the Michael addition is performed at a mild temperature, for instance at 40°C. To achieve virtually complete conversion, rather long reaction times are applied, for instance 12 hours.

The process according to the invention offers the advantage that the vinyl cyanide can be added to the reaction mixture in one go. Moreover, there is no necessity of cooling the reactor to prevent undesired side reactions.

The pressure during the Michael addition reaction is not critical. The Michael addition is mostly performed at atmospheric pressure, but can also be performed at elevated pressure.

The Michael addition at atmospheric pressure is mostly carried out at a temperature between 60 and 100°C, preferably between 70 and 90°C. When the Michael addition is carried out at elevated pressure, higher temperatures can be applied.

It has been found that in the process according to the invention the reaction time can be reduced considerably. Surprisingly, it has been found that in spite of the application of a higher temperature the selectivity of the Michael addition is enhanced.

It appears that the time within which virtually full conversion is achieved decreases with increasing vinyl cyanide concentration. It appeared to be possible to obtain virtually full conversion in the Michael addition according to the invention in a time which is for instance shorter than 8 hours or shorter than 5 hours. The reaction time of the Michael addition is mostly longer than 0.5 hour.

Optionally, a catalyst can be added to the reaction mixture in the Michael addition in order to enable the reaction of the functional groups with the vinyl cyanide units to run well. Examples of catalysts suitable for this purpose are weak acids, such as acetic acid, or (weak) bases. The amount of catalyst added to the reaction mixture is mostly 0-5 mol% relative to the number of reactive sites R.

Usually, the cyanide-terminated reaction product of the Michael addition can be isolated in a simple manner, for instance by distilling off the excess of nitrile and part of the solvent and subsequently effecting a phase separation between, on the one hand, the rest of the solvent with a minor quantity of nitrile and, on the other, the dendrimer with some water. To that end, first the excess of nitrile is distilled off at a temperature preferably lower than 80°C and then the reaction mixture is cooled to a temperature between room temperature and 60°C in order to achieve an optimum phase separation. Preferably, the mixture is cooled to a temperature between room temperature and 45°C. The solvent layer which does not contain the dendrimer contains any by-products and non-reacted vinyl cyanide and can be re-used in a subsequent Michael addition reaction.

The purity of the cyanide-terminated reaction product obtained by the process according to the invention appears to be higher than the purity of the product obtained by the process described in WO-A-9314147. In the process according to the invention an additional purification step, such as for instance recrystallization, is possible, but mostly superfluous. In the process according to the invention, the cyanide-terminated reaction product is mostly obtained with a selectivity higher than 99%. The selectivity per addition of vinylcyanide unit is preferably higher than or equal to 99.5%.

The cyanide group of the cyanide-terminated reaction product thus obtained is subsequently reduced to an amine group by means of a hydrogenation reaction. If the incorporated vinyl cyanide unit is acrylonitrile, a propylamine (PA) unit is thus formed.

With the process according to the invention it appeared to be possible to realize a virtually complete hydrogenation of nitrile groups in dendrimers with more than 8, in particular more than 10 nitrile groups.

The process according to the invention appears to be particularly suitable for the hydrogenation of nitriles in dendritic macromolecules with more than 10, in particular more than 15 nitrile groups.

The solvent used in the hydrogenation according to the invention is an alcohol which contains an amount of ammonia, the molar ratio between ammonia and the number of cyanide groups being higher than 0.8. In order to achieve a good selectivity, it appears that the molar ratio between the amount of ammonia and the number of cyanide groups should increase with increasing number of generation. A good selectivity in the hydrogenation reaction is achieved if the molar ratio between ammonia and the number of cyanide groups is lower than 50 or lower than 20.

Suitable alcohols are for instance low-boiling alcohols, such as for instance methanol, ethanol, isopropanol. Optionally, a mixture of different alcohols is used. The alcohol is preferably methanol. Optionally, a mixture of one or more alcohols and water is used. The water : alcohol weight ratio is mostly between 1:50 and 2:1. The water : alcohol weight ratio preferably is between 1:10 and 1:1.

Surprisingly, it has been found that with the hydrogenation according to the invention it appears to be possible to increase the selectivity of a subsequent Michael addition reaction from about 95% to 99.5% or higher.

The hydrogenation reaction can be effected for instance by reacting the incorporated cyanide groups with H₂ gas. If complete reduction is desired the molar ratio between the H₂ and the cyanide groups should be large enough. Mostly, a molar ratio of at least 2 is used.

The hydrogenation step is carried out in the presence of a suitable catalyst. In general a hydrogenation catalyst is used, preferably a heterogeneous hydrogenation catalyst.

The catalyst used according to the invention comprises a metal from group VIII of the periodic system of the elements as it is represented on the cover of the Handbook of Chemistry and Physics, 58th edition, CRC Press, 1977-1978. It is known that metals from group VIII show activity in the hydrogenation of nitriles. See for this for instance EP-A-0077911. Well suited are nickel, cobalt, platinum, palladium and rhodium. For good catalytic activity, the catalyst preferably has a large active metal surface area. The metal can be used as such or supported on a suitable carrier.

Particularly suitable as catalyst according to the invention are Raney nickel or Raney cobalt. Such catalysts are well known in the art, and for instance described in US-A-1628190.

Raney nickel consists mainly of nickel and aluminium, the latter in the form of metallic aluminium, aluminium oxides and/or aluminium hydroxides. Minor quantities of other metals, such as iron and/or chromium, can be added in elementary or bonded form to the Raney nickel in order to enhance the activity and selectivity in the hydrogenation of certain groups of compounds. It is known that Raney nickel catalysts promoted with iron and/or chromium are especially suitable for the reduction of nitrile groups; see for instance S.R. Montgomery, Catalysis of Organic Reactions 5, pp. 383-409 (1981).

Raney cobalt also contains aluminium and may be provided with promoters. It is known for instance that Raney cobalt promoted with chromium is suitable for the hydrogenation of nitriles.

Optionally, the catalyst can be washed, for instance with the solvent of the hydrogenation reaction, with an alcohol, with a mixture of different alcohols or a mixture of water and one or more alcohols. Suitable alcohols are for instance methanol, ethanol, isopropanol.

The maximum amount of catalyst that can be used in the reactor in the hydrogenation of the cyanide-terminated product depends on the type of reactor that is used. It will be easy for one skilled in the art to determine the suitable amount of catalyst for any desired reactor.

Surprisingly, it has been found that in the process according to the invention it is possible to substantially lower the catalyst concentration and at the same time achieve a considerable improvement in the efficiency of utilization of the capacity of the reactors.

In the process according to the state of the art catalyst concentrations between 100 and 400% relative to the weight of the substrate are applied.

In the process according to the invention the required amount of catalyst seems to increase with increasing dendrimer generation. In the process according to the invention the weight ratio of the required amount of catalyst (dry) relative to the amount of dendritic macromolecule is mostly larger than 10%. The required amount of catalyst (dry) relative to the weight of the cyanide-terminated dendritic macromolecule is preferably larger than 12% and smaller than 50%.

The process according to the invention has the advantage that the amount of cyanide-terminated dendrimer that can be hydrogenated per volume unit can be increased by at least a factor of 5 compared with the process described in WO-A-9314147. In the process described in WO-A-9314147, usually 1.5-4.5 wt.% of cyanide-terminated substrate is hydrogenated. In the process according to the present invention the amount of cyanide-terminated product that can be hydrogenated is mostly higher than 10 wt.%. Often even higher than 20 wt.%, relative to the total weight of the reaction mixture.

The hydrogenation reaction can be carried out for instance in a closed reactor under a H₂ atmosphere. The total pressure prevailing in the reactor is mainly caused by the hydrogen and ammonia present at a certain temperature and is mostly between 1 and 500 bar, preferably between 10 and 200 bar, most preferably between 10 and 100 bar. The hydrogen pressure prevailing in the reactor is mostly higher than 1, preferably higher than 10 bar.

The reaction temperature is not critical and is mostly between 0 and 200°C, preferably between 10 and 150°C, more preferably between 50 and 110°C. After the hydrogenation reaction a product is obtained which contains terminal amine groups.

The hydrogenation according to the invention offers the advantage that foaming can be avoided during the hydrogenation reaction, upon opening of the reactor, or upon termination of the hydrogenation reaction.

When the hydrogenation reaction is completed the catalyst can be removed from the reaction mixture. This can be effected for instance by cooling the reactor under a H₂ atmosphere and, after the H₂ has been discharged, purging the reactor with inert gas and filtering off the reactor contents. The filtrate contains the dendrimers in solution.

It is also possible to mount a so-called 'filter candle' (a filter made of sintered metal) in the reactor. The filtrate is then removed from the reactor via the inner space of the filter, while the catalyst remains on the outside of the filter in the reactor. The advantage of this procedure is that the reactor can be kept under pressure, which permits to carry out several successive hydrogenation reactions whereby hydrogen deficiency of the catalyst is avoided.

The process according to the invention also allows the use of regenerated catalysts. A used catalyst can for example be regenerated by treating it for several hours with a caustic solution, for instance an aqueous NaOH solution, at a temperature between 50 and 70°C. After filtering off, the catalyst is subsequently washed with deionized water until the washing water has an approximately neutral pH. The catalyst is stored under water.

The molecular weight of the dendritic macromolecule obtained is mostly higher than 800, in particular higher than 1500; mostly it is lower than 100,000, in particular lower than 50,000.

The dendritic macromolecule obtained can optionally be wholly or partially modified with a variety of functional groups. This can be effected for instance by allowing the available amine or nitrile groups, optionally in the presence of a suitable catalyst, to react wholly or partially with suitable reagents. Examples of such reagents are α,β-unsaturated compounds substituted with electron-withdrawing groups, unsaturated aliphatic esters and amides, such as for instance acrylic ester, methacrylic ester, crotylic ester and acrylamide, polyamides such as nylon 4,6, nylon 6, nylon 6,10, nylon 8, epoxides such as ethylene oxide and propylene oxide, acid halides, such as for instance acid chlorides, acryloyl chloride, alkyl halides, such as for instance epichlorohydrine, ethyl bromoacetate and allyl bromide, aryl halides, such as for instance benzyl chloride, tosyl halides, such as for instance tosyl chloride, anhydrides, such as for instance phthalic anhydride, dicarboxylic acids, such as for instance terephthalic acid and adipic acid, diols, (a)cyclic aldehydes such as formaldehyde, acetaldehyde, hexanal, benzaldehyde, pyridine aldehydes, p-formyl phenyl acetic acid and 1,4,5,8-naphthalene tetraacetaldehyde, ketones, such as for instance derivatized cyclohexanones (e.g. HALS compounds), lactide, lactones, such as for instance caprolactone, phosphate esters as described in US-A-3,855,364, molecules with a chiral centre. This enumeration is not exhaustive and therefore non-restrictive.

The invention also relates to a process for the purification of amine terminated dendrimers that contain impurities, for example one or more metals and/or metal ions, for example cobalt or nickel. Most often these metals and/or metal ions originate from the catalyst. The presence of cobalt for example not only appears to have a negative effect on the colour of the dendritic macromolecule, but also on the thermal stability of the dendritic macromolecule.

According to the invention, the metals and/or metal ions can be removed from a dendrimer by washing the dendrimer with a solution of a salt forming acid. According to this process, the dendrimer remains in solution whereas the metal salt precipitates. An example of a suitable solution is carbondioxide containing water. If necessary, the mixture can be heated, whereby the temperature will usually be kept lower than 150°C. The mixture is preferably heated at a temperature below 100°C, more preferably between 50 and 70°C. In case carbon dioxide containing water is applied, cobalt ions for example precipitate as cobalt carbonate. The cobalt carbonate can be separated from the dendrimer solution for example by filtration.

According to the invention, the metal ions can also be removed by submitting the amine terminated metal containing dendrimer to a second treatment with hydrogen. This method is particularly suitable for the removal of metals and/or metal ions from dendrimers of the higher generations, such as dendrimers of the 4^{th} or 5^{th} generation or higher. Usually, the metal containing dendrimers are dissolved in a suitable solvent. The dendrimer solution is submitted to a second treatment with hydrogen by applying a hydrogen pressure as described before, at moderate temperature. Usually, the temperature is kept below 200°, preferably below 150°C, more preferably below 100°C. If necessary in order to further decrease the metals and/or metal ion content of the dendrimer, the hydrogen treatment can be repeated a third time. In case of cobalt containing dendrimers, cobalt ions for example precipitate as metallic cobalt, which can be removed by filtration.

Suitable solvents are for instance low boiling alcohols such as for instance methanol, ethanol, isopropanol and mixtures of these alcohols. Preferably, methanol is used.

The invention will be further elucidated by means of the following examples without being restricted thereto.

### Example I

1200 ml water and 150 g (1.7 mol) of 1,4-diaminobutane (DAB, substrate) were introduced into a three-neck 2-litre flask provided with a stirrer. 400 g of pure acrylonitrile (ACN) were added in one go to this mixture. The resulting reaction mixture was heated at 80°C for 1 hour.

Next, both the water and the excess of acrylonitrile were evaporated under reduced pressure at a temperature of 50°C. HPLC analysis of the resulting residue showed that >99% pure product had been obtained without additional recrystallization of said residue. Recrystallization of the residue in methanol gave no further improvement of the purity of the product.

The isolated product was analyzed by means of ¹H-NMR and ¹³C-NMR and mass spectrometry, which revealed that the product obtained was DAB(ACN)₄.
¹³C NMR (50 MHz D₂O): 119 ppm, CN; 53.1 ppm, NCH₂(CH₂)₃; 49.4 ppm, NCH₂CH₂CN; 24.9 ppm, NCH₂CH₂CN; 16.9 ppm CH₂CN.
¹H NMR (200 MHz, CDCl₃): 2.85 ppm, t, 2H, NCH₂CH₂CN; 2.55 ppm, m, 1H, NCH₂(CH2)₃; 2.48 ppm, t, 2H, CH₂CN; 1.55 ppm, m, 1H, CH₂CH₂N.

### Example II

5.6 g of Raney cobalt catalyst (wet) (type Grace 2724 from Grace; manufacturer's specifications: 78-96 wt.% Co, 0.5-5 wt.% Cr, 0.5-5 wt.% Ni, 3-12 wt.% Al) were washed once with 25 ml of ethanol at a temperature of 20°C. Then the catalyst was introduced into an autoclave and methanol was added to a total weight of 52.71 g of methanol. Finally, about 22.7 g of DAB(ACN)₄ (7 wt.% water) in powder form were added. 12.3 wt% of dry catalyst relative to DAB(ACN)₄ was added.

When the autoclave had been closed, stirring of the mixture was started and the autoclave was purged three times with N₂ gas and three times with H₂ gas. After letting off the pressure, about 6.3 g of liquid ammonia were supplied to the autoclave. The molar ratio between the ammonia and DAB(ACN)₄ was about 4.9. Next, the autoclave was heated with stirring to 65°C at a H₂ pressure of 80 bar.

The reaction was stopped after 20 minutes and the autoclave was cooled under H₂ to room temperature. Then the H₂ was drained, the autoclave was purged three times with N₂ gas and opened, upon which its contents were immediately filtered off.

The isolated product was analyzed by means of ¹³C NMR spectroscopy, which revealed that the product obtained was 1,4-diaminobutane-N,N'-tetra-1-propylamine, DAB(PA)₄. The conversion was virtually complete.
¹³C NMR (50 MHz D₂O): 53.4 ppm, NCH₂CH₂CH₂CH₂ (2x); 51.1 ppm, NCH₂CH₂CH₂NH₂ (4x); 39.5 ppm, CH₂NH₂ (4x); 28.8 ppm, CH₂CH₂NH₂ (4x); 23.9 ppm, NCH₂CH₂CH₂CH₂N (2x).

### Example III

Example I was repeated, using 5.0 g of DAB(PA)₄ as substrate instead of 1,4-diaminobutane. To this mixture, 20.67 g of ACN were added in one go. The reaction mixture thus obtained was heated at 80°C for 2 hours.

The isolated product was analyzed by means of ¹³C NMR spectroscopy, which revealed that the product obtained was DAB(PA)₄(ACN)₈. The yield was >99.7%.
¹³C NMR (50 MHz, CDCl₃): 118.9 ppm, CN (8x); 53.9 ppm, NCH₂CH₂CH₂CH₂ (2x); 51.5 and 51.4 ppm, NCH₂CH₂CH₂N (8x); 49.6 ppm, NCH₂CH₂CN (8x); 25,0 and 24,9 ppm, NCH₂CH₂CH₂CH₂ and NCH₂CH₂CH₂N (6x); 16,9 ppm, CH₂CN (8x).

### Example IV

Example II was repeated, with 11.23 g of Raney cobalt catalyst (wet) being washed once with about 25 ml of methanol at a temperature of 20°C and then introduced into the autoclave, after which methanol was added to a total weight of 46.87 g of methanol. Finally, about 28.2 g of DAB(PA)₄(ACN)₈ (containing 20 wt.% of water) were added. About 24.9% of catalyst relative to DAB(PA)₄(ACN)₈ was added. About 4.5 g of liquid ammonia were introduced into the autoclave. The molar ratio between ammonia and DAB(PA)₄(ACN)₈ was about 8.7. DAB(PA)₄(ACN)₈ was reduced to DAB(PA)₄(PA)₈ in 200 minutes at 46°C and a H₂ pressure of 80 bar.

The isolated product was analyzed by means of ¹³C NMR spectroscopy, which revealed that the product obtained was DAB(PA)₄(PA)₈.
¹³C NMR (50 MHz, D₂O): 53.6 ppm, NCH₂CH₂CH₂CH₂ (2x); 51.7 ppm, NCH₂CH₂CH₂N (8x); 51.2 ppm, NCH₂CH₂CH₂NH₂ (8x); 39.6 ppm CH₂NH₂ (8x); 28.9 ppm, CH₂CH₂NH₂ (8x); 24.1 ppm, NCH₂CH₂CH₂CH₂N (2x); 22.3 ppm, NCH₂CH₂CH₂N (4x).

### Example V

Example I was repeated but now 17.71 g of DAB(PA)₄(PA)₈ were used as substrate instead of DAB. To this mixture, 41.34 g of ACN were added in one go. The reaction mixture thus obtained was heated at 80°C for 3 hours.

The isolated product was analyzed by means of ¹³C NMR spectroscopy, which revealed that the product obtained was DAB(PA)₄(PA)₈(ACN)₁₆.
¹³C NMR (50 MHz, CDCl₃): 119.0 ppm, CN (16x); 54.1 ppm, NCH₂CH₂CH₂CH₂ (2x); 52.2 ppm, NCH₂CH₂CH₂ (8x); 51.5 en 51.4 ppm, NCH₂CH₂CH₂ (16x); 49.5 ppm, NCH₂CH₂CN (16x); 25.0 en 24.9 ppm NCH₂CH₂CH₂CH₂ and NCH₂CH₂CH₂N (10x); 24.3 ppm, NCH₂CH₂CH₂N (4x); 16.9 ppm, CH₂CN (16x).

### Example VI

Example II was repeated, 20.86 g of DAB(PA)₄(PA)₈(ACN)₁₆ (containing 18.3 wt.% of water) being reduced at 80°C for 240 minutes in the presence of 17.63 g of Raney cobalt catalyst (wet), 54.1 g of methanol and 27.3 g of ammonia. About 51.8% catalyst (dry) relative to DAB(PA)₄(PA)₈(ACN)₁₆ was added. Ammonia was added in a molar ratio of 152.6 relative to DAB(PA)₄(PA)₈(ACN)₁₆. The isolated product was analyzed by means of ¹³C NMR spectroscopy, which revealed that the product obtained was DAB(PA)₄(PA)₈(PA)₁₆.
¹³C NMR (50 MHz, D₂O): 53.6 ppm, NCH₂CH₂CH₂CH₂ (2x); 51.7 ppm, NCH₂CH₂CH₂N (24x); 51.2 ppm, NCH₂CH₂CH₂NH₂ (16x); 39.6 ppm, CH₂NH₂ (16x); 28.9 ppm, CH₂CH₂NH₂ (16x); 24.1 ppm, NCH₂CH₂CH₂CH₂N (2x); 22.3 ppm, NCH₂CH₂CH₂N (12x).

### Example VII

Example I was repeated, but now 38.7 g of DAB(PA)₄(PA)₈(PA)₁₆ were used as substrate instead of DAB. To this mixture, 83 g of ACN were added in one go. The reaction mixture thus obtained was heated at 80°C for 4 hours.

The isolated product was analyzed by means of ¹³C NMR spectroscopy, which revealed that the product obtained was DAB(PA)₄(PA)₈(PA)₁₆(ACN)₃₂.
¹³C NMR (50 MHz, CDCl₃): 119.0 ppm, CN (32x); 54.2 ppm, NCH₂CH₂CH₂CH₂ (2x); 52.2 ppm, NCH₂CH₂CH₂ (24x); 51.4 ppm, NCH₂CH₂CH₂ (32x); 49.4 ppm, NCH₂CH₂CN (32x); 24.9 ppm NCH₂CH₂CH₂CH₂ and NCH₂CH₂CH₂N (18x); 24.4 ppm, NCH₂CH₂CH₂N (12x); 16.8 ppm, CH₂CN (32x).

### Example VIII

Example II was repeated, 14.2 g of DAB(PA)₄(PA)₈(PA)₁₆(ACN)₃₂ (containing 14.1 wt.% of water) being reduced at 80°C for 360 minutes in the presence of 11.39 g of Raney cobalt catalyst (wet), 34.9 g of ammonia and 1.03 g of water. About 46.9% catalyst (dry) relative to DAB(PA)₄(PA)₈(PA)₁₆(ACN)₃₂ was added. Ammonia was added in a molar ratio of 568.9 relative to DAB(PA)₄(PA)₈(PA)₁₆(ACN)_{32.} The isolated product was analyzed by means of ¹³C NMR spectroscopy, which revealed that the product obtained was DAB(PA)₄(PA)₈(PA)₁₆(PA)₃₂.
¹³C NMR (50 MHz, D₂O): 51.7 ppm, NCH₂CH₂CH₂N (56x); 51.2 ppm, NCH₂CH₂CH₂NH₂ (32x); 39.6 ppm, CH₂NH₂ (32x); 28.8 ppm, CH₂CH₂NH₂ (32x); 22.3 ppm, NCH₂CH₂CH₂N (28x).

### Example IX Regenerated catalyst

Example II was repeated, but instead of fresh catalyst, the same quantity of catalyst regenerated with NaOH was used. About 24 g of Raney cobalt which had been used one time in the hydrogenation of cyanide-terminated product was suspended in a three-neck flask containing 175 ml of a 5% NaOH solution. After stirring for 2 hours at 60°C and subsequent cooling to room temperature, the catalyst was filtered off and washed with deionized water until the pH of the washing water was about 7. Next, the regenerated catalyst was used in the hydrogenation of DAB(ACN)₄ as described in example II. Full conversion was achieved.

### Example X-XII

### Purification of 3^{rd}, 4^{th} and 5^{th} generation dendrimers

100g of a 25% solution of DAB(PA)ₙ was loaded into a reactor wiht a content of 160 ml. After purging the reactor with nitrogen three times and three times with hydrogen, the hydrogen pressure is raised to 800 psi (50 bar). The temperature in the reactor was increased to 100°C while stirring. Thereafter, the hydrogen pressure is raised to 1200 psi (80 bar) for the times indicated in table 1.

The reactor was then cooled to room temperature and subsequently purged with nitrogen three times. After opening the reactor, a few grams of Celite 521® were added and the solution was filtered off. The filtrate, which contained the dendrimer, was evaporated in a rotavapor. The cobalt content of the dendrimers was determined using X-ray fluorescence spectroscopy. The results are shown in table 1.

**Table 1**

| Type | temperature (°C) | time (h) | initial Co content (ppm) | Co content after hydrogenation (ppm) |
|---|---|---|---|---|
| DAB(PA)₁₆ | 100 | 3 | 40 | 5 |
| DAB(PA)₃₂ | 100 | 3 | 256 | 5 |
| DAB(PA)₆₄ | 100 | 3 | 650 | 15 |

## Claims

1. Process for the preparation of a dendritic macromolecule, wherein an amount of a core molecule comprising at least one functional group is dissolved in a solvent, after which alternatingly an addition reaction and a hydrogenation reaction are carried out, during the addition reaction a compound of formula 1: 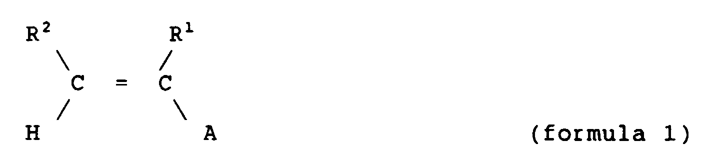 where
R¹ = -H or -CH₃;
R² = -H, -CH₃, or a hydrocarbon compound having 2-18 carbon atoms and containing at least one double bond conjugated with the double bond of formula 1;
A = -C≡N, 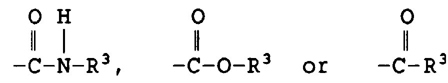
R³ = a hydrocarbon compound having 1-18 carbon atoms and containing 1-5 cyanide groups;
being added to the solution which reacts with the functional groups in such a manner that a dendritic macromolecule with terminal cyanide groups is formed, and during the hydrogenation reaction the cyanide groups being reduced in solution by means of hydrogen and a suitable catalyst in such a manner that functional amine groups are formed, characterized in that the solvent in which the hydrogenation reaction takes place is an alcohol which contains an amount of ammonia, the molar ratio between the amount of ammonia and the number of cyanide groups being higher than 0.8.

2. Process according to claim 1, characterized in that the molar ratio between the amount of ammonia and the number of cyanide groups is lower than 50.

3. Process according to any one of claims 1-2, characterized in that the alcohol is methanol.

4. Process according to any one of claims 1-3, characterized in that the alcohol contains water.

5. Process according to claim 4, characterized in that the alcohol : water weight ratio is between 1:50 and 2:1.

6. Process according to any one of claims 1-5, characterized in that the catalyst for the hydrogenation reaction is Raney nickel or Raney cobalt.

7. Process according to claim 6, characterized in that the catalyst is regenerated by washing with a caustic solution.

8. Process according to any one of claims 1-7, characterized in that the weight ratio of the amount of catalyst relative to the amount of cyanide-terminated dendrimer in the hydrogenation reaction is smaller than 50%.

9. Process according to any one of claims 1-8, characterized in that the core molecule is a polymethylene diamine.

10. Process according to any one of claims 1-8, characterized in that the core molecule is a dendritic macromolecule.

11. Process according to any one of claims 1-10, characterized in that the compound of formula 1: 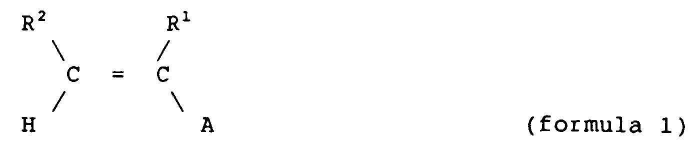 where
R¹ = -H or -CH₃;
R² = -H, -CH₃, or a hydrocarbon compound having 2-18 carbon atoms and containing at least one double bond conjugated with the double bond of formula 1;
A = -C≡N, 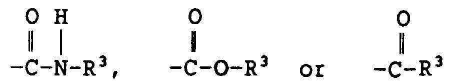
R³ = a hydrocarbon compound having 1-18 carbon atoms and containing 1-5 cyanide groups;
is acrylonitrile.

12. Process according to any one of claims 1-11, characterized in that the Michael addition is carried out in water as solvent.

13. Process according to any one of claims 1-12, characterized in that the Michael addition is carried out at a temperature between 60 and 100°C.

14. Process for isolation of a cyanide-terminated product obtained with the process according to any one of claims 1-13, characterized in that the reaction mixture is cooled to a temperature between room temperature and 60°C, after which the cyanide-terminated product is isolated through phase separation.

15. Process for the purification of a dendritic macromolecule obtained with the process according to any one of the claims 1-14, characterized in that the amine terminated dendrimer is submitted to a hydrogen treatment.

16. Process for the purification of a dendritic macromolecule obtained with the process according to any one of the claims 1-14, characterized in that the amine terminated dendrimer is washed with carbondioxide containing water.

## Patentansprüche

1. Verfahren zur Herstellung eines dendritischen Makromoleküls, bei welchem eine Menge eines Kernmoleküls, das zumindest eine funktionelle Gruppe umfaßt, in einem Lösungsmittel gelöst wird, wonach abwechselnd eine Additionsreaktion und eine Hydrierungsreaktion durchgeführt werden, wobei während der Additionsreaktion eine Verbindung der Formel (1): 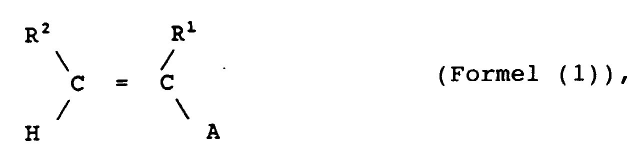 worin
R¹ = -H oder -CH₃;
R² = -H, -CH₃ oder eine Kohlenwasserstoff-Verbindung mit 2 bis 18 Kohlenstoffatomen und mit zumindest einer Doppelbindung, die mit der Doppelbindung der Formel (1) konjugiert ist;
A = -C≡N, 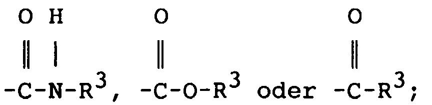
R³ = eine Kohlenwasserstoff-Verbindung mit 1 bis 18 Kohlenstoffatomen und mit 1 bis 5 Cyanid-Gruppen;
der Lösung zugesetzt wird, die mit den funktionellen Gruppen derart reagiert, daß ein dendritisches Makromolekül mit terminalen Cyanid-Gruppen gebildet wird, und während der Hydrierungsreaktion die Cyanid-Gruppen in Lösung mit Wasserstoff und einem geeigneten Katalysator derart reduziert werden, daß funktionelle Amin-Gruppen gebildet werden, dadurch gekennzeichnet, daß das Lösungsmittel, in dem die Hydrierungsreaktion stattfindet, ein Alkohol ist, der eine Menge an Ammoniak enthält, wobei das Molverhältnis zwischen der Menge an Ammoniak und der Anzahl von Cyanid-Gruppen höher ist als 0,8.

2. Verfahren nach Anspruch 1, dadurch gekennzeichnet, daß das Molverhältnis zwischen der Menge an Ammoniak und der Anzahl von Cyanid-Gruppen niedriger ist als 50.

3. Verfahren nach einem der Ansprüche 1 und 2, dadurch gekennzeichnet, daß der Alkohol Methanol ist.

4. Verfahren nach einem der Ansprüche 1 bis 3, dadurch gekennzeichnet, daß der Alkohol Wasser enthält.

5. Verfahren nach Anspruch 4, dadurch gekennzeichnet, daß das Alkohol:Wasser-Masseverhältnis zwischen 1:50 und 2:1 beträgt.

6. Verfahren nach einem der Ansprüche 1 bis 5, dadurch gekennzeichnet, daß der Katalysator für die Hydrierungsreaktion Raney-Nickel oder Raney-Kobalt ist.

7. Verfahren nach Anspruch 6, dadurch gekennzeichnet, daß der Katalysator durch Waschen mit einer Ätzlösung regeneriert wird.

8. Verfahren nach einem der Ansprüche 1 bis 7, dadurch gekennzeichnet, daß das Masseverhältnis der Menge an Katalysator, bezogen auf die Menge an Dendrimer mit terminalem Cyanid, in der Hydrierungsreaktion weniger als 50 % beträgt.

9. Verfahren nach einem der Ansprüche 1 bis 8, dadurch gekennzeichnet, daß das Kernmolekül ein Polymethylendiamin ist.

10. Verfahren nach einem der Ansprüche 1 bis 8, dadurch gekennzeichnet, daß das Kernmolekül ein dendritisches Makromolekül ist.

11. Verfahren nach einem der Ansprüche 1 bis 10, dadurch gekennzeichnet, daß die Verbindung der Formel (1): 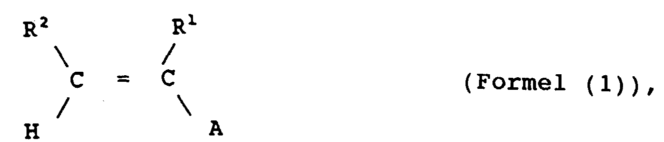 worin
R¹ = -H oder -CH₃;
R² = -H, -CH₃ oder eine Kohlenwasserstoff-Verbindung mit 2 bis 18 Kohlenstoffatomen und mit zumindest einer Doppelbindung, die mit der Doppelbindung der Formel (1) konjugiert ist;
A = -C≡N, 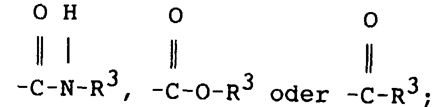
R³ = eine Kohlenwasserstoff-Verbindung mit 1 bis 18 Kohlenstoffatomen und mit 1 bis 5 Cyanid-Gruppen;
Acrylnitril ist.

12. Verfahren nach einem der Ansprüche 1 bis 11, dadurch gekennzeichnet, daß die Michael-Addition in Wasser als Lösungsmittel durchgeführt wird.

13. Verfahren nach einem der Ansprüche 1 bis 12, dadurch gekennzeichnet, daß die Michael-Addition bei einer Temperatur zwischen 60 und 100°C durchgeführt wird.

14. Verfahren zur Isolierung eines Produkts mit terminalem Cyanid, das durch das Verfahren nach einem der Ansprüche 1 bis 13 erhalten wird, dadurch gekennzeichnet, daß die Reaktionsmischung auf eine Temperatur zwischen Raumtemperatur und 60°C abgekühlt wird, wonach das Produkt mit terminalem Cyanid durch Phasentrennung isoliert wird.

15. Verfahren zur Reinigung eines dendritischen Makromoleküls, das mit dem Verfahren nach einem der Ansprüche 1 bis 14 erhalten wird, dadurch gekennzeichnet, daß das Dendrimer mit terminalem Amin einer Wasserstoff-Behandlung unterzogen wird.

16. Verfahren zur Reinigung eines dendritischen Makromoleküls, das mit dem Verfahren nach einem der Ansprüche 1 bis 14 erhalten wird, dadurch gekennzeichnet, daß das Dendrimer mit terminalem Amin mit Kohlendioxid enthaltendem Wasser gewaschen wird.

## Revendications

1. Procédé de préparation d'une macromolécule dendritique où on dissout une certaine quantité de molécule de coeur comprenant au moins un groupe fonctionnel dans un solvant, après quoi on réalise alternativement une réaction d'addition et une réaction d'hydrogénation, pendant la réaction d'addition d'un composé de formule I : 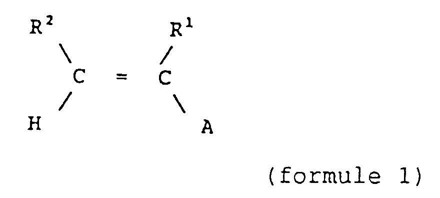 où
R¹ = -H ou -CH₃ ;
R² = -H, -CH₃, ou un composé hydrocarbure ayant 2 - 18 atomes de carbone et contenant au moins une double liaison conjuguée avec la double liaison de formule 1 ;
A = -C≡N, 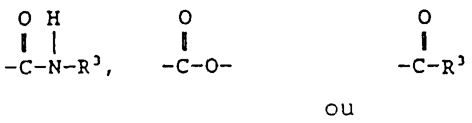 ou
R³ = un composé hydrocarbure ayant 1 - 18 atomes de carbone et contenant 1 - 5 groupes cyanure ;
est ajouté à la solution qui réagit avec les groupes fonctionnels de telle manière que l'on forme une macromolécule dendritique avec des groupes cyanure terminaux, et pendant la réaction d'hydrogénation, les groupes cyanure sont réduits en solution au moyen d'hydrogène et un catalyseur approprié, de telle manière que l'on forme des groupes fonctionnels amine, caractérisé en ce que le solvant où la réaction d'hydrogénation a lieu est un alcool qui contient une certaine quantité d'ammoniaque, le rapport molaire entre la quantité d'ammoniaque et le nombre de groupes cyanure étant supérieur à 0,8.

2. Procédé selon la revendication 1, caractérisé en ce que le rapport molaire entre la quantité d'ammoniaque et le nombre de groupes cyanure est inférieur à 50.

3. Procédé selon l'une quelconque des revendications 1 - 2, caractérisé en ce que l'alcool est le méthanol.

4. Procédé selon l'une quelconque des revendications 1 - 3, caractérisé en ce que l'alcool contient de l'eau.

5. Procédé selon la revendication 4, caractérisé en ce que le rapport pondéral alcool : eau est compris entre 1 : 50 et 2 : 1.

6. Procédé selon l'une quelconque des revendications 1 - 5, caractérisé en ce que le catalyseur de la réaction d'hydrogénation est du nickel Raney ou du cobalt Raney.

7. Procédé selon la revendication 6, caractérisé en ce que le catalyseur est régénéré par lavage avec une solution caustique.

8. Procédé selon l'une quelconque des revendications 1 - 7, caractérisé en ce que le rapport pondéral de la quantité de catalyseur par rapport à la quantité de dendrimère à terminaison cyanure dans la réaction d'hydrogénation est inférieur à 50%.

9. Procédé selon l'une quelconque des revendications 1 - 8, caractérisé en ce que la molécule de coeur est une polyméthylènediamine.

10. Procédé selon l'une quelconque des revendications 1 - 8, caractérisé en ce que la molécule de coeur est une macromolécule dendritique.

11. Procédé selon l'une quelconque des revendications 1 - 10, caractérisé en ce que le composé de formule 1 : 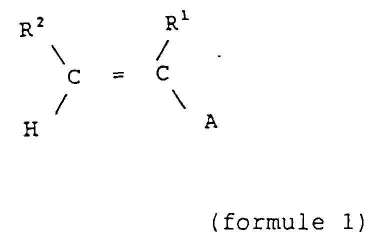 où
R¹ = -H ou -CH₃ ;
R² = -H, -CH₃, ou un composé hydrocarbure ayant 2 - 18 atomes de carbone et contenant au moins une double liaison conjuguée avec la double liaison de formule 1 ;
A = -C≡N, 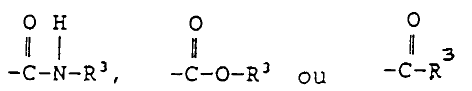
R³ = un composé hydrocarbure ayant 1 - 18 atomes de carbone et contenant 1 - 5 groupes cyanure ;
est l'acrylonitrile.

12. Procédé selon l'une quelconque des revendications 1 - 11, caractérisé en ce que l'on réalise la réaction d'addition de Michael dans l'eau comme solvant.

13. Procédé selon l'une quelconque des revendications 1 - 12, caractérisé en ce que l'on réalise la réaction d'addition de Michael à une température comprise entre 60 et 100°C.

14. Procédé d'isolement d'un produit à terminaison cyanure obtenu par le procédé selon l'une quelconque des revendications 1 - 13, caractérisé en ce que l'on refroidit le mélange réactionnel à une température comprise entre la température ambiante et 60°C, après quoi on isole le produit à terminaison cyanure par séparation de phase.

15. Procédé de purification d'une macromolécule dendritique obtenue avec le procédé selon l'une quelconque des revendications 1 - 14, caractérisé en ce que le dendrimère à terminaison amine est soumis à un traitement par l'hydrogène.

16. Procédé de purification d'une macromolécule dendritique obtenue par le procédé selon l'une quelconque des revendications 1 - 14, caractérisé en ce qu'on lave le dendrimère à terminaison amine avec de l'eau contenant du dioxyde de carbone.
